# EUROPEAN PATENT SPECIFICATION

(11) **EP 1 365 713 B1**
(45) Date of publication and mention of the grant of the patent: **26.10.2005**
(21) Application number: 02712576.4
(22) Date of filing: 18.02.2002
(51) Int. Cl.: A61F 5/48

(54) **URINE INCONTINENCE DEVICE**
HARNINKONTINENZVORRICHTUNG
DISPOSITIF D'INCONTINENCE D'URINE

(30) Priority: 19.02.2001 SE 0100538
(43) Date of publication of application: 03.12.2003
(73) Proprietor: Uriplug Aktiebolag, 223 50 Lund (SE)
(72) Inventor: CLAREN, Jan, 223 50 Lund (SE); MATTIASSON, Anders, 247 91 Södra Sandby (SE)
(74) Representative: Thiel, Christian
(86) International application number: PCT/SE2002/000279
(87) International publication number: WO 2002/065959

(56) References cited:
- DE-A1- 4 206 857
- US-A- 4 361 150
- US-A- 5 671 755

## Description

### FIELD OF THE INVENTION

The present invention relates to devices for use in urine incontinence, including a means for insertion into the urethra and the blocking thereof to prevent urine from passing the means. Devices of this kind are used to prevent urine leakage and unintended urination in men.

### BACKGROUND OF THE INVENTION

There exist a great number of devices of various kind and design for coping with urine incontinence. Several of these devices are designed to be put on the penis; thereby urine is collected or urination is prevented. Such a kind of device is designed to clamp the urethra to thereby prevent leakage of urine.

There is also a number of devices for coping with urine incontinence of which at least a portion are designed for insertion into the urethra. An device of this sort is shown and described in WO 97/13480. The device described in WO 97/13480 comprises a cover to be threaded on the glans penis. The cover is provided with a protruding portion for insertion into the urethra. When the user applies the cover onto the penis the protruding portion is inserted into the urethra and blocks it. The portion designed for insertion into the urethra thus is intended to prevent urine leakage by blocking the urethra in a way to prevent urine from passing.

US 5671755 A discloses an assembly for prevention of urinary incontinence in humans comprising an applicator and an expandable body comprising an appendage. The applicator is adapted to position the body in the urethra of a person. When the person wishes to urinate the body can be withdrawn by the appendage. Such withdrawal may however cause irritation in the terminal portion of the urethra since the body is removed in an expanded condition. The need for an appendage which extends from the mouth of the urethra is also problematic for hygienic and safety reasons.

Except for the device disclosed in US 5671755. A drawback with such devices for mounting on or insertion into the penis is that they may cause discomfort to the bearer since they are comparatively clumsy and affect the mobility of the bearer. A further drawback is that these devices are not so easy to use, in particular for elderly persons in whom incontinence is prevalent. In addition known devices for coping with urine incontinence may unintentionally glide off the penis, thus giving rise to urine leakage. Still another drawback are skin irritations which can arise due to portions abutting the penis. Also, there is a comparatively high risk of infection since the majority of known device are intended for repeated use. This problem is particularly evident with device intended for insertion into the urethra. A further drawback with these devices is that the blood flow in the penis and to the penis may be hindered which necessitates the removal of the device in regular intervals to restore normal blood flow to the penis.

Another major problem with the majority of device for coping with urine incontinence known in the art is that they do not take into consideration differences between individuals. Such differences may, for instance, exist in regard of dimensions, thereby varying the fit of device intended for mounting on the penis or for insertion into the penis. This may result in reduced safety in regard of protection against incontinence or in other discomfort to the user. Consequently certain of these devices have to be adapted individually which is thought to result in high costs.

A general problem with these devices is that they can only be used for coping with male incontinence for anatomic reasons.

### OBJECTS OF THE INVENTION

It is an object of the present invention to provide a device of the aforementioned kind, which is safe, easy to use, does not cause discomfort to the patient, and is cheap to produce, in particular if made disposable.

It is another object of the invention to provide a device, which does not need to be adapted individually due to anatomical differences between the sexes and individual persons.

Further objects of the invention will be evident from the following short description of the invention, a number of figures illustrating preferred embodiments thereof, which are described in more detail, and the appended claims.

### SUMMARY OF THE INVENTION

According to the present invention is disclosed a device comprising a means in form of a first composition comprising a gel enclosed in a first container. The container is provided with a mouthpiece and an outfeed means by which the first composition can be expelled from the container and fed into a urethra.

It should be kept in mind that the container, the mouthpiece and the outfeed means can be designed in various ways and are not limited to the embodiments described herein. For instance, the container may be somewhat oblong and designed in a manner so as to be generally circular in cross section. The container may also be somewhat conical in form at the portion connecting it with the mouthpiece. Thus the container tapers off in the direction of the mouthpiece to facilitate pressing the composition out from the container and into the mouthpiece by means of the outfeed means.

The mouthpiece is connected to the remainder of the container in a manner so as to enable the first composition to be fed out from the container through the mouthpiece by the outfeed means. The mouthpiece is suitably formed as an oblong tube generally circular in cross section and protruding from the container in a longitudinal direction thereof. Suitably the mouthpiece is designed in a somewhat conical manner so as to make the portion connecting the mouthpiece and the container having a somewhat larger diameter than the free end of the mouthpiece. The mouthpiece thus is designed for insertion into a urethra and for transport of the first composition from the container into the urethra.

Suitably the outfeed means is disposed at aside of the container facing away from the mouthpiece. The outfeed means can take the form of an device for spraying out or pumping out the first composition in a conventional manner, whereby the composition can be fed out continuously or in given doses. The outfeed means can also be designed with a means for dosage control, whereby the doses can be adapted individually.

The first composition is designed for transport from a first container to a portion in the urethra suitable for blocking. For instance, the first composition can be inserted about 10 cm into the urethra. The first composition comprises a gel or is capable of forming a gel when leaving the container or when abutting the urethra. Upon abutting the urethra the first composition exerts a pressure against the inner face of the urethra while a plug in form of a sphere or similar form for blocking of the urethra is being formed. The first composition preferably consists of tissue compatible components. The gel may be made expandable by, for instance, incorporation of a low boiling solvent, such as pentane or cyclopentane, or a suitable gas, such as butane or carbon dioxide, or a composition capable of forming such gas.

The invention may also comprise a second composition enclosed in a second container. The second container may be designed in a manner corresponding to the first container. The second composition is designed for removal of the urethra blockage caused by the first composition. Thus the second composition is designed for being fed into the urethra and for dissolving the first composition upon coming in contact with the latter to allow the user to urinate without constraint. Residues of the first and second compositions, if present, are washed out when urinating, the urethra thereby being restored to its natural state.

In case of, incontinence, in particular of male incontinence, the first composition may also be designed in a manner to allow removal of the urethra blockage by physical working thereof. By such physical working, for instance by working the outside of the penis, the blockage in form of the first composition is deformed and eventually pressed out of the urethra, whereby the blockage of the urethra is removed. Portions of the first composition possibly left in the urethra are washed out at urination.

The first composition may comprise a composition of a chemical or biochemical nature, such as polysaccharide gels based on, for instance, agarose, dextrin, starch, cellulose and cellulose derivatives, such as hydroxyethyl cellulose, hydroxypropyl cellulose, methyl cellulose, ethyl cellulose, hydroxyethyl-methyl cellulose, hydroxyethyl-ethyl cellulose, hydroxypropyl-methyl cellulose, chitosan, xantan, alginate and derivatives thereof. Alternatively the first composition may comprise acrylate gels based on, for instance, acrylamide and its derivatives. The first composition may also be yielding, ductile, and biocompatible, may comprise bacteriostatic or bacteriocidal means, and may, by itself, constitute a barrier to bacteria. Furthermore the first composition may comprise properties promoting mucosal growth and thus may be designed in the form of a wound gel.

The second composition may comprise a chemical mixture or solution comprising, for instance, a salt or a combination of salts, which counteracts the expanding (swelling) properties of the first composition and/or dissolves or degrades physically and/or chemically the first composition to remove the blockage formed by the first composition.

In addition the second composition may have pH which, in contact with the first composition, counteracts the expanding (swelling) properties of the first composition. The second composition may also consist of various liquids the properties of which, such as surface tension, hydrophilicity, and viscosity can counteract the expanding properties of the first composition. It is also within the reach of the present invention to combine agents having the aforementioned properties for removal of the plug formed by the first composition.

According to a first preferred aspect of the present invention is disclosed a first device containing a first urethral plug forming composition of the aforementioned kind and a second device containing second urethral plug removing composition, said devices being of the aforementioned kind. It is preferred for the mouthpiece openings of the devices to be sealed by a removable seal, in particular a seal that cannot be applied again; thereby the devices are made obligatorily disposable and cannot cause infection of the urethra.

According to a second preferred aspect of the invention the gel of the invention may be formed in situ by the reaction of two first composition components which are simultaneously and/or consecutively injected into the urethra, in particular by a device similar to that described above but having two chambers instead of one, each chamber holding one first composition component. The chambers are sealed prior to use to prevent the first composition components from mixing. After removal of the seal the first composition components are injected into the urethra in which they mix to form a first composition, which quickly turns to an expanding polymer plug; mixing may also occur in the distal portion of the device during or immediately prior to first composition component expulsion. For instance, one first composition component may contain the solution of a monomer, oligomer or pre-polymer whereas the other may contain a polymerization catalyst in solution. Alternatively one of the first composition components may be a polymer whereas the other first composition component may be a compound capable of reversibly reacting with said one of the first composition components, such as a monomer, oligomer or polymer possessing suitable reactive groups. Certain useful components may, for instance, be sensitive to heat and polymerize or condense at body temperature; in such case they have to be injected in at a temperature substantially below the body temperature. Temperature sensitive polymers for use in the invention are poly(N-vinyl amides), poly(N-alkyl acrylamides), poly(alkylene oxides) as well their random, block and graft copolymers with other monomers. Other useful components include pH-sensitive polymers which are soluble in a highly charged state but form gels if the pH is adjusted. Examples for this group are co-polymers of acrylic acid, metacrylic acid, hyaluronic acid and chitosan. Useful systems incorporating two components comprise poly(vinylalcohol) and borate/boronate containing compounds, polymers capable having a ligand capable of interaction with a polymer of a different kind. An example for such systems is one comprising a polymer having chelating groups complexed by two- or multivalent metal ions and a histidine rich polymer or the couple polymer carbohydrate/lectin or a polymer with antigen moieties linked thereto capable of binding to antibodies linked to another polymer or to free antibodies. Combinations of these systems are also within the scope of the present invention such as, for instance, a precipitation induced by a change in temperature followed by reaction of the precipitated polymer with another polymer.

According to a third preferred aspect of the invention are disclosed corresponding suitable first and second compositions.

### SHORT DESCRIPTION OF THE DRAWINGS

The invention will now be explained in more detail by reference to drawings, in which
Fig. 1 is a general view, in a longitudinal section, of a first composition enclosed in a first container, illustrating a first embodiment of the container;
Fig. 2 is a general view, in a longitudinal section, of a first composition enclosed in a first container, illustrating a second embodiment of the invention;
Fig. 3 is a partial view, in a longitudinal section, of the insertion of the device of Fig. 2 into an urethra of a male person, after expulsion of the first composition and formation of a plug, the device not being shown in section;
Fig. 4 is a partial view, in a longitudinal section, of the insertion of a device corresponding to the device of Fig. 2 but filled with a second composition, after expulsion of the second composition and its action on the urethral plug formed by the first composition, the device not being shown in section;
Fig. 5 is a general view of a third embodiment of the invention with two chambers filled with a first and a second fluid capable of forming a first composition on mixing, in a longitudinal section.

### DESCRIPTION OF PREFERRED EMBODIMENTS

### EXAMPLE 1. Devices

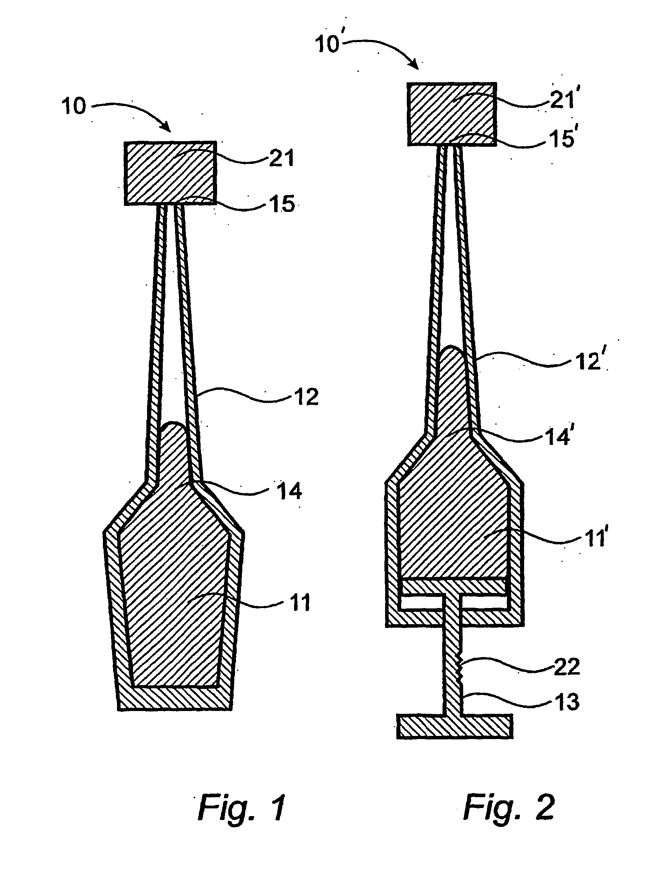

By reference to the Figures, in particular Fig. 1 and Fig. 2, is illustrated a first embodiment of the device according to the invention comprising a first container 10 comprising a mouthpiece 12 and holding a first plug forming composition 11.

It should be noted that thickness dimensions of materials, distances between various components, and other geometric relationships are not to scale in the Figures. Furthermore the container 10, in particular, may be designed in various ways and is in no way limited by the embodiments described herein. Preferably the container 10 is made of a flexible polymer material, such as polyethylene or polypropylene.

The container 10 of Fig. 1 is oblong and has a generally circular cross section; it is closed at its one (proximal) end and open at its other (distal) end. Its rear portion forms a first opening 14 at which it merges with the wall of the mouthpiece portion 12. By its viscosity the first composition 11 is essentially kept in the rear portion of the first container 10. The rear portion of the container 10 is designed biconically to facilitate the transfer of the first composition 11 to the mouthpiece 12. Thus the rear portion of the container 10 tapers off towards the first opening 14. The mouthpiece 12 protrudes distally from the rear portion in the longitudinal direction of container 10, forming a narrowed extension of a generally circular cross section. At its free (distal) end the mouthpiece 12 comprises a second opening 15, through which the first composition 11 can be expelled from the container 10.

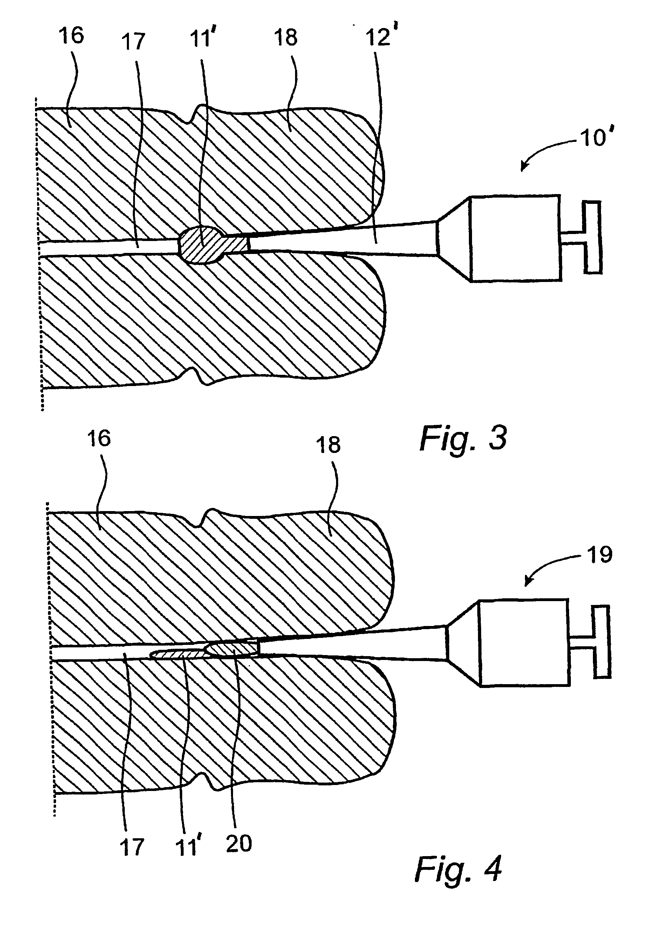

The mouthpiece 12 tapers off in the direction of the second opening 15. The mouthpiece 12 is designed for partial insertion into the urethra; see, Figs. 3 and 4.
The second opening of the mouthpiece 12 is preferably provided with a seal 21 to keep the first composition 11 sterile and to prevent the first composition from leaving the container 10 prior to use. The seal 21 is arranged in a manner so at to make the seal 21 removable prior to the application of the first composition 11. The seal 21 can be designed in any conventional manner. It is intended to be removed before applying the first composition. For instance, the seal 21 is integrated with the mouthpiece 12 and is removed by tearing it off. The seal 21 may also take the form of a distal end portion integral with the mouth piece 12 and joined to the latter at a circular kerf; in such case the opening 15 is formed by breaking off said distal end portion.

The first composition 11 in the container 10 shown in Fig. 1 can be expelled by compressing the container 10. The container 10 holds an amount of the first composition 11 suitable for at minimum a single administration. The container 10 may be designed as a disposable package.

The second embodiment of the container 10 shown in Fig. 2 in which the same reference figures are used for elements corresponding to those of the embodiment of Fig. 1 comprises an outfeed means 13 in form of a piston. The outfeed means 13 is disposed in the cylindrical rear (proximal) portion of the container 10' in a manner corresponding to that of a piston in a syringe for injection. By pushing the pusher of the piston 13 in a distal direction the first composition 11' is expelled from the container 10'. It may be fed out continuously or in selected doses, which can be determined by a means of a marking 22 applied on the piston 13 or on the container wall which, in such case, has to be transparent. The dosing can be adapted individually according to the desire of the user.

Now referring also to Fig. 3, the mouthpiece 12' of the first container 10' is disposed in the end portion of a urethra 17 in a penis 16. The disposition of the mouthpiece is that required for feeding the first composition 11'' to a correct position in the urethra 17. The mouthpiece 12 is inserted into the urethra 17 via the mouth of the urethra at the glans 18 penis 16. Usually insertion to a depth of about 10 mm will suffice. In Fig. 3 is shown a state in which a portion of the first composition 11' has been expelled from the container 10' and a plug 20 has been formed. Upon feeding the first composition 11' into the urethra the mouthpiece 12' is removed from the urethra 17 (not shown).

The first composition 11' is placed in the urethra at a certain depth for blocking the urethra. Thus the first composition 11' abuts the inner face of the urethra 17. By gel formation and, possibly, expansion of the first composition 11', the first composition 11 presses against the inner face of the urethra 17, whereby the first composition 11' can assume a generally spherical form. The abutment of the first composition 11' against the inner face of the urethra 17 and the expanding properties of the first composition 11' result in the blocking of the urethra 17 which prevents urine from passing the first composition 11.

The first composition may comprise chemical compositions such as polysaccharide gels based on, for instance, agarose, dextrin, starch, cellulose, alginate, xanthan or chitosan and derivatives thereof. Alternatively the first composition 11 may comprise acrylate gels based on, for instance, acrylamide or derivatives thereof. In a preferred embodiment the first composition is an alginate gel. Suitably the first composition is yielding, ductile, and biocompatible, and may comprise bacteriostatic agents, which can form a barrier against bacteria. Furthermore the first composition may comprise properties that promote growth of the urethral mucosa.

A further embodiment of the present invention is shown in Fig. 4. A second container 19 containing a second composition 20 is arranged for feeding the second composition 20 into the urethra 17. The second container 19 can be designed similar to the first container 10, and thus comprises a mouthpiece 12'' and an outfeed means such as described above in connection with the first container 10.

The second composition 20 is designed for being fed into the urethra 17. Thus the second composition is fed into the urethra at or near the blockage to bring it in contact with the latter, whereby the blockage is removed by dissolution of the plug formed by the first composition 11'. Alternatively the second composition 20 may comprise properties, such as shrinkage inducing properties, which oppose the expanding or gel stabilizing properties of the first composition 11', whereby the blockage can be removed after shrinking of the plug. The second composition may comprise a chemical composition of a combination of various salts. Furthermore the second composition 20 may have a pH-value which, in contact with the first composition 11', counteracts the plug retaining properties of the first composition 11'. The second composition 20 may also consist of various solvents of which properties like surface tension, hydrophilicity, and viscosity can counteract the expanding or gel stabilizing properties of the first composition 11'.

Thus the second composition 20 is designed for being fed into the urethra 17 and to contact the first composition. These results in the first composition 11' being dissolved or degraded or diminished in size by shrinkage, which allows the user to urinate if so desired. On urinating rests of the first and second compositions 11', if any, 20 are removed, whereby the urethra is reconstituted.

In an embodiment of the present invention the first composition 11,11' can also be designed in a manner to let the blockage of the urethra 17 caused by the first composition 11,11' be removed by certain physical working. By this physical working, in particular of the outside of the penis 16, the blockage in form of the first composition 11,11' is pressed out, whereby it changes its form so that the blockage of the urethra is removed. When urinating the first composition 11,11' is then driven out from the urethra 17 by the urine.

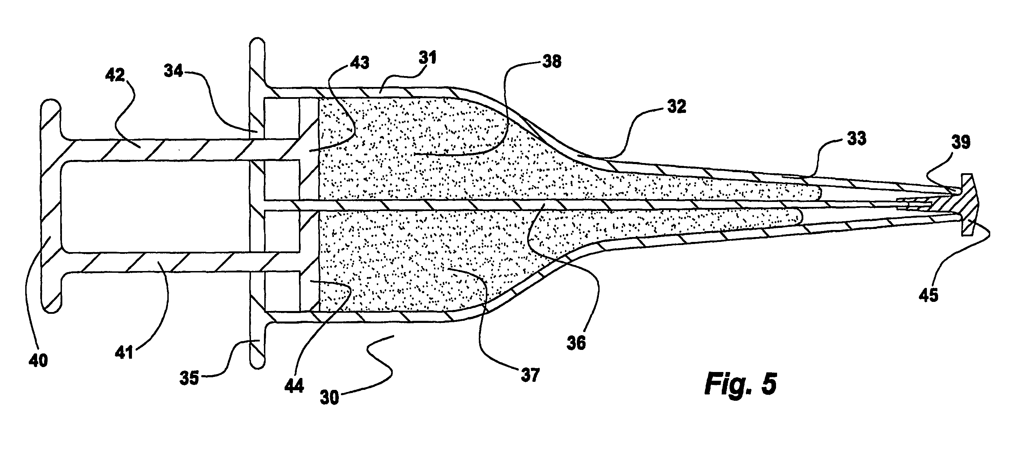

The embodiment shown in Fig. 5 is a device 30 according to the invention provided with first and a second chambers containing a first fluid 37 and a second fluid 38. The fluids 37,38 contain components which are capable of forming a first gelatinous composition on mixing. The first and second chambers are enclosed by a common rotationally symmetric wall 31 extending from a circular rear wall 34 in a distal direction in which the outer diameter gradually diminishes over an intermediate wall section 32 which to which a mouthpiece section 33 ending in a mouthpiece 39 is joined. The rear wall 34 is provided with a peripheral circular flange 35 and is integral with wall sections 31, 32, 33 and a flat inner wall 36 disposed between the first and second chambers. The inner wall 36 does not extend to the mouthpiece 39 but ends a little proximally of it. The distal end of the inner wall 36 is capable of being received in a proximal slit of a sealing stopper 45, which seals the first and second chambers from each other as well as the mouthpiece 39 and is removed prior to use. The first and second fluids 37,38 are expelled from the device 30 at the same rate by first 44,41 and second 43,42 plungers, respectively, the rods 41,42 of which are sealingly (not shown) guided in openings of the rear wall 34 and are joined by a transverse pusher 40 at their proximal end. This provides for their concerted displacement when pushed in a distal direction to expulse the first and second fluids 37,38 from the respective chambers. As explained for the other embodiments the mouthpiece 39 can be inserted into an urethra after removal of the stopper 45 into which the first and second fluids can be injected simultaneously. By making the inner wall 36 end at a distance proximal of the mouthpiece opening a terminal space is formed in the mouthpiece 39 adjacent to said opening in which the first and second fluids partially or fully mix before leaving the mouthpiece. Mixing can be improved by, for instance, arranging mechanical means, which make the flow to become turbulent, in the terminal space, such as ribs disposed on the inner wall of the space (not shown). As is evident from the foregoing the first and second chambers and the plunger heads 44,43 are semicircular in a transverse section.

### EXAMPLE 2. Compositions

A preferred embodiment of the first composition is based on the complex formation between boronate groups and polyol compounds. Poly(vinyl alcohol) (PVA) has been used as a polyol, and copolymers of and N-acryloyl-p-aminophenylboronic acid as a source of boronate groups, the following called boronate polymer. The complex formation between two polymers of this kind results in gel formation. A preferred embodiment of the second composition comprises glucose. Glucose competes with PVA for boronate groups. This results in the boronate-PVA bonds responsible for the attraction between two polymers being broken, and in the dissolution of the free polymers thus formed.

The model polymer system of the first composition forms is capable of quickly forming a gel which is sufficiently dense to stay in the urethra for extended periods of time, such as one hour or more, thereby occluding the urethra and preventing leakage of urine. On the other hand the gel plug formed by the first composition is relatively fast dissolved on addition of glucose or another suitable low-molecular-weight saccharide. The gel formation of poly(vinyl alcohol) (PVA) with borate polymer proceeds at a high rate at alkaline conditions (pH >9.5), whereas the dissolution of the gel proceeds at a high rate in a solution containing 1% by weight of glucose at pH 3. The PVA-gel of the invention was formed within seconds after mixing an aqueous PVA solution with an aqueous borate polymer solution, and was dense enough to prevent water flow through a tube at a pressure of about 50 cm of water column. The gel dissolved within 10-15 min when immersed in 1 % glucose solution, pH 3.

Instead of gel formation with boronate polymer the effect of 0.1M Na₂B₄O₇ (pH 9.8) on aqueous PVA solutions of varying content was studied. A PVA concentration of 3% w/v was sufficient for gel formation, while the lower concentrations (≤2%) did not give a gel or a visually noticeable increase in solution viscosity. Decimolar sodium tetraborate (pH 9.8) or its mixtures with 0.4 M boric acid (pH varying from 7.1 to 8.5, 0.5 ml) were added to a 5% w/v aqueous solution of PVA (0.5 ml) in a vial immediately followed by shaking to mix the solutions. Gelation was visually estimated by turning the vials upside down 1 min, 3 h, and 24 h after mixing. Efficiency of gelation was designated as follows: - (no gelation), -/+ (incomplete gel formation; separation of the mixture into phases with lower and higher viscosity) +/- (quick gel formation followed by gradual collapse of the gel and appearance of a liquid phase in a few hours), + (stable gel after 24 h). The results are summarized in Table 1.

**Table 1.**

| *Gelation of boronate buffer solutions with PVA as a function of pH* | | | |
|---|---|---|---|
| pH 7.1 | pH 7.8 | pH 8.5 | pH 9.8 |
| - | -/+ | + | + |

As follows from Table 1 efficient gelation of boronate buffers with PVA is possible in basic media, where a certain share of boronate ions is present in charged tetrahedral forms. Lowering of boron concentration from 0.4 to 0.2 moles/l led to a weaker gel-formation (-/+ type) at pH 8.5. 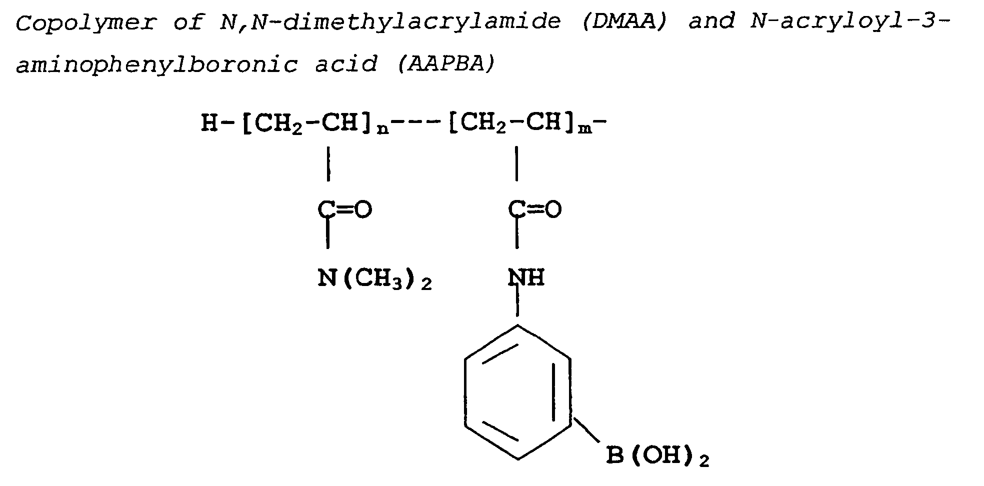 *Synthesis of AAPBA*. 3-aminophenylboronic acid hydrochloride (3-APBA) (1.72 g, 10 mmol) was dissolved in 20 ml 2M NaOH (40 mmol). The solution was filtered and cooled to 3° C in an ice bath. Acryloylchloride (1.6 ml, 20 mmol) was added drop-wise to the solution of 3-APBA while intensively stirring for 5 - 6 min. HCl (2 M) was added drop-wise to the reaction mixture to adjust its pH to about 1. The precipitate was separated by filtration on a sintered glass filter (Schott, Duran, No.3) and washed with distilled water (50 ml). The washed precipitate was dissolved in 40 ml distilled water at 60°C from which it crystallized overnight at 8°C as light-violet needles. The crystals were filtered off, washed with cold distilled water and dried under vacuum in a desiccator over CaCl₂. The yield of AAPBA was 0.76 g (40%). ¹H NMR (CD₃SOCD₃): δ = 5.73 (1H, a), 6.25 (1H, b), 6.45 (1H, c), 7.2 - 7.9 (4H, d), 8.0 (2H, e), 10.05 (1H, f).

*Boronate-containing polymers.* Two samples termed pac-B1 and pac-B2 were prepared by free-radical copolymerization of DMAA and AAPBA. Commercial DMAA was separated from hydroquinone monomethyl ether (polymerization inhibitor) by flash chromatography on a 0.9 x 2.5 cm column packed with dry activated aluminum oxide; 3 ml of the starting material gave 1.9 ml of pure DMAA.

*Pac-B1*. DMAA (0.93 ml, 9 mmol) was dissolved in 5 ml of dioxane. AAPBA (190 mg, 1 mmol) was dissolved in 4 ml of dioxane by heating to 70°C. The monomer solutions were combined in a beaker and mixed, followed by dissolving 20 mg of 2,2'-azobis(2-methylpropionitrile) (AIBN) in the mixture. Free radical polymerization was started by heating the mixture under N₂ to 70°C for 30 min. The gelatinous copolymer formed was dissolved in 8 ml of ethanol, and the solution was added drop-wise to 100 ml of diethyl ether to precipitate the copolymer. The precipitate was collected by filtration, washed with diethyl ether and dried under vacuum; yield 0.40 g (37%).

*Pac-B2*. DMAA (0.93 ml, 9 mmol), AAPBA (190 mg, 1 mmol) and AIBN (10 mg) were dissolved in 10 ml of ethanol. Free radical polymerization was started by heating the reaction mixture under N₂ to 70°C and keeping it at that temperature for 6 h. The thus obtained solution of copolymer was added drop-wise to 100 ml diethyl ether to precipitate the copolymer. The precipitate was collected by filtration, washed with diethyl ether and dried under vacuum; yield 0.87 g (77%).

The pac-B1 and -B2 polymers were characterized by ¹H NMR, and the molar fractions of AAPBA-units, m/(m+n), were quantified as 12 mol% and 8 mol%, respectively. The calculation was performed according to formula: m/(m+n) = (d+e)/(c+d+e) or m/(m+n) = (d+e)/6 : [(d+e)/6 + a/2] which gave similar results.

Although the copolymers were closely related in regard of their chemical composition, their solubility in water was found to be substantially different: pac-B2 easily dissolved in distilled water at any pH, while pac-B1 was only soluble under basic conditions. Presumably, the higher content of AAPBA units makes the PAC-B1 copolymer more hydrophobic so that it could only be dissolved if its boronate functions became partially charged at higher pH.

Gelation of PVA with pac-B1 or pac-B2. Equal volumes of the solutions were mixed at various pH. Results are summarized in Table 2.

**Table 2.**

| *Gelation of the boronate-containing copolymers with PVA as a function of pH* | | | | |
|---|---|---|---|---|
| Sample | PH 5.7 | PH 7.0 | PH8.1 | PH 8.5 |
| Pac-B1 | Not soluble | Not soluble | 2%: -/+ | 1%: + 2%: + or +/- |
| Pac-B2 | 1%: - 2%: +/- | 1%: - 2%: +/- | 1%: - 2%: +/- | 1%: - 2%: + or +/- |

As follows from Tables 1 and 2, pac-B2 is capable of gelation with PVA over a much wider range of pH compared to the low molecular weight boronates. The underlying reason is probably a multipoint binding of the copolymers to PVA. If at least two charged boronate functions belong to a single polymeric chain, it may react as a macromolecular crosslinker of PVA.

It is worth to note that gelation of PVA with the boronate-containing copolymer proceeds at a much lower boron concentration in the solution compared to low molecular weight boronates: 0.015 g-atom/l (2%w/v pac-B2) against 0.4 g-atom/l (0.1M Na₂B₄O₇ + 0.4M H₃BO₃, 1:1, pH 8.5). This is another evidence for the efficient macromolecular crosslinking of PVA by the boronate-containing copolymers. The gels formed were easily dissolved in 1 % glucose solution, pH 3.

## Claims

1. A device for use in urine incontinence, comprising a means for insertion into a urethra (17), whereby the urethra (17) is blocked, the means comprising a first composition (11;11') being enclosed in a first container (10;10') provided with a mouthpiece (12;12'), the mouthpiece (12;12') being designed for insertion into the urethra (17) and for transport of the first composition (11;11') from the first container (10;10') into the urethra (17),
**characterized in that**
the first composition (11;11') comprises a gel or is capable of forming a gel when injected into an urethra (17) for the blockage thereof.

2. The device according to claim 1, wherein the first container (10) is made of a flexible material, whereby the first composition (11) can be expelled from the container by compression of the container.

3. The device according to claim 1, wherein the first container (10') comprises an outfeed means (13) arranged for feeding out the first composition (11') enclosed in the first container (10').

4. The device according to claim 3, wherein the outfeed means (13) is designed to allow given doses to be fed out of the first composition (11').

5. The device according to claim 1, wherein the first composition (11;11') is arranged deformably for removal of urethral blockage by physical working.

6. A device for use in urine incontinence, comprising a means for insertion into a urethra (17), the means comprising a second composition (20) being enclosed in a second container (19) provided with a mouthpiece (12''), the mouthpiece (12'') being designed for insertion into the urethra (17) and for transport of the second composition (20) from the second container (19) into the urethra (17),
**characterized in that**
the second composition (20) comprises an agent for removing the urethral blockage caused by the gel of the first composition (11') of claim 1.

7. The device according to claim 6, wherein the second composition (20) is designed for counteracting a property of the first composition (11;11').

8. The device according to claim 7, wherein the second container (19) comprises a mouthpiece (12'') and a removable seal to allow the second composition (20) to be fed out of the second container (19) into an urethra (17) upon inserting the mouthpiece (12'') of the second container (19) into said urethra (17), by compression of the second container or by a separate outfeed means.

9. The device according to claim 1, wherein the first composition (11) comprises bacteriocidal or bacteriostatic agents or the composition as such constitutes a barrier against bacteria in the urethra.

10. The device according to claim 1, wherein the first composition (11) comprises an agent or a combination of agents promoting mucosal growth in the urethra.

11. Device according to claim 1, wherein the first composition comprises a polysaccharide gel based on, for instance, agarose, dextrin, starch, cellulose and derivatives thereof, including hydroxyethyl cellulose, hydroxypropyl cellulose, methyl cellulose, ethyl cellulose, hydroxyethyl-methyl cellulose, hydroxyethyl-ethyl cellulose, hydroxypropyl-methyl cellulose, and chitosan and their derivatives, acrylate gels and derivatives thereof, and xanthan gels and derivatives thereof.

12. Device according to claim 8, wherein the second composition (20) comprises an agent and/or a solvent and/or a salt which has a pH-value capable of removing the blockage effected by the first composition (11).

## Patentansprüche

1. Vorrichtung zur Anwendung bei Harninkontinenz, umfassend ein Mittel zum Einführen in eine Harnröhre (17), wodurch die Harnröhre (17) blockiert wird, wobei das Mittel umfasst eine erste Zusammensetzung (11, 11'), die in einem ersten Behälter (10; 10') eingeschlossen ist, der mit einem Mundstück (12; 12') versehen ist, wobei das Mundstück (12; 12') zum Einführen in die Harnröhre (17) und zum Transport der ersten Zusammensetzung (11; 11') aus dem ersten Behälter (10; 10') in die Harnröhre (17) ausgebildet ist,
**dadurch gekennzeichnet ist, dass**
die erste Zusammensetzung (11; 11') ein Gel umfasst oder in der Lage ist, ein Gel zu bilden, wenn sie zu deren Blockierung in die Harnröhre (17) eingeführt wird.

2. Vorrichtung nach Anspruch 1, wobei der erste Behälter (10) aus einem flexiblen Material hergestellt ist, wodurch die erste Zusammensetzung (11) aus dem Behälter durch Zusammendrücken des Behälters ausgestoßen werden kann.

3. Vorrichtung nach Anspruch 1, wobei der erste Behälter (10') ein Auslassmittel (13) umfasst, dass zum Ausbringen der ersten Zusammensetzung (11'), die in dem ersten Behälter (10') eingeschlossen ist, ausgebildet ist.

4. Vorrichtung nach Anspruch 3, wobei das Auslassmittel (13) so ausgebildet ist, um das Ausbringen einer gegebenen Dosis der ersten Zusammensetzung (11') zu ermöglichen

5. Vorrichtung nach Anspruch 1, wobei die erste Zusammensetzung (11, 11') verformbar ausgebildet ist zur Entfernung der Harnröhren-Blockade durch physikalisches Bearbeiten.

6. Vorrichtung zur Anwendung bei Harninkontinenz, umfassend ein Mittel zum Einführen in eine Harnröhre (17), wobei das Mittel eine zweite Zusammensetzung (20) umfasst, die in einem zweiten Behälter (19) eingeschlossen ist, der mit einem Mundstück (12") versehen ist, wobei das Mundstück (12") zum Einführen in die Harnröhre (17) und zum Transport der zweiten Zusammensetzung (20) aus dem zweiten Behälter (19) in die Harnröhre (17) ausgebildet ist,
**dadurch gekennzeichnet ist, dass**
die zweite Zusammensetzung (20) ein Mittel zum Entfernen der Harnröhren-Blockade enthält, die durch das Gel der ersten Zusammensetzung (11') nach Anspruch 1 hervorgerufen worden ist.

7. Vorrichtung nach Anspruch 6, wobei die zweite Zusammensetzung (20) so ausgebildet ist, dass sie einer Eigenschaft der ersten Zusammensetzung (11, 11') entgegen wirkt.

8. Vorrichtung nach Anspruch 7, wobei der zweite Behälter (19) ein Mundstück (12") und eine entfernbare Abdichtung umfasst, um es zu ermöglichen, dass die zweite Zusammensetzung (20) aus dem zweiten Behälter (19) durch Zusammendrücken des zweiten Behälters oder durch ein getrenntes Auslassmittel in die Harnröhre (17) ausgebracht wird, nachdem das Mundstück (12") des zweiten Behälters (19) in die Harnröhre (17) eingeführt worden ist.

9. Vorrichtung nach Anspruch 1, wobei die erste Zusammensetzung (11) bakterizide oder bakteriestatische Mittel umfasst oder die Zusammensetzung als solche eine Barriere gegen Bakterien in der Harnröhre bildet.

10. Vorrichtung nach Anspruch 1, wobei die erste Zusammensetzung (11) ein Mittel oder eine Kombination von Mitteln umfasst, das/die das Wachstum der Schleimhaut in der Harnröhre fördert.

11. Vorrichtung nach Anspruch 1, wobei die erste Zusammensetzung ein Polysaccharid-Gel, z.B. auf Basis von Agarose, Dextrin, Stärke, Cellulose und Derivate davon, umfassend Hydroxyethyl-cellulose, Hydroxypropyl-cellulose, Methyl-cellulose, Ethyl-cellulose, Hydroxyethyl-methyl-cellulose, Hydroxyethyl-ethyl-cellulose, Hydroxypropyl-methyl-ethyl-cellulose, und Chitosan und seine Derivate, Acrylat-Gele und Derivate davon und Xanthan-Gele und Derivate davon umfasst.

12. Vorrichtung nach Anspruch 8, wobei die zweite Zusammensetzung (20) ein Mittel und/oder ein Lösungsmittel und/oder ein Salz umfasst, das einen pH-Wert hat, der die durch die erste Zusammensetzung (11) hervorgerufene Blockade entfernt.

## Revendications

1. Dispositif destiné à une utilisation en liaison avec une incontinence urinaire, comprenant un moyen en vue d'une insertion dans un urètre **(17),** l'urètre **(17)** étant ainsi bloqué, le moyen comprenant une première composition **(11 ; 11')** contenue dans un premier récipient **(10 ; 10')** muni d'un embout **(12 ; 12')**, l'embout **(12 ; 12')** étant conçu en vue d'une insertion dans l'urètre **(17)** et en vue du transport de la première composition **(11 ; 11')** du premier récipient **(10 ; 10')** dans l'urètre **(17),**
**caractérisé en ce que** la première composition **(11 ; 11')** comprend un gel ou est capable de former un gel une fois injectée dans un urètre **(17)** en vue du blocage de celui-ci.

2. Dispositif selon la revendication 1, dans lequel le premier récipient **(10)** est constitué d'un matériau flexible, de sorte que la première composition **(11)** peut être éjectée du récipient par compression du récipient.

3. Dispositif selon la revendication 1, dans lequel le premier récipient **(10')** comprend un moyen de sortie **(13)** agencé pour faire sortir la première composition **(11')** contenue dans le premier récipient **(10')**.

4. Dispositif selon la revendication 3, dans lequel le moyen de sortie **(13)** est conçu pour permettre de faire sortir des doses données de la première composition **(11')**.

5. Dispositif selon la revendication 1, dans lequel la première composition **(11 ; 11')** est agencée, de façon déformable, en vue du retrait du blocage de l'urètre par une action physique.

6. Dispositif destiné à une utilisation en liaison avec une incontinence urinaire, comprenant un moyen en vue d'une insertion dans un urètre **(17)**, le moyen comprenant une deuxième composition **(20)** contenue dans un deuxième récipient **(19)** muni d'un embout **(12'')**, l'embout **(12'')** étant conçu en vue d'une insertion dans l'urètre **(17)** et en vue du transport de la deuxième composition **(20)** du deuxième récipient **(19)** dans l'urètre **(17),**
**caractérisé en ce que** la deuxième composition **(20)** comprend un agent pour retirer le blocage de l'urètre provoqué par le gel de la première composition **(11')** de la revendication 1.

7. Dispositif selon la revendication 6, dans lequel la deuxième composition **(20)** est conçue pour annihiler une propriété de la première composition **(11 ; 11')**.

8. Dispositif selon la revendication 7, dans lequel le deuxième récipient **(19)** comprend un embout **(12'')** et un scellement amovible pour permettre à la deuxième composition **(20)** d'être extraite du deuxième récipient **(19)** dans l'urètre **(17)** après insertion de l'embout **(12'')** du deuxième récipient **(19)** dans ledit urètre **(17)**, par compression du deuxième récipient ou par un moyen de sortie séparé.

9. Dispositif selon la revendication 1, dans lequel la première composition **(11)** comprend des agents bactéricides ou bactériostatiques ou la composition en tant que telle constitue une barrière contre les bactéries dans l'urètre.

10. Dispositif selon la revendication 1, dans lequel la première composition **(11)** comprend un agent ou une combinaison d'agents favorisant la croissance des muqueuses dans l'urètre.

11. Dispositif selon la revendication 1, dans lequel la première composition comprend un gel de poylsaccharide à base de, par exemple, agarose, dextrine, amidon, cellulose et leurs dérivés, notamment hydroxyéthyl cellulose, hydroxypropyl cellulose, méthyl cellulose, éthyl cellulose, hydroxyéthyl-méthyl cellulose, hydroxyéthyl cellulose, hydroxypropy-méthyl cellulose, chitosane et leurs dérivés, des gels d'acrylate et leurs dérivés, et des gels de xanthane et leurs dérivés.

12. Dispositif selon la revendication 8, dans lequel la deuxième composition **(20)** comprend un agent et/ou un solvant et/ou un sel qui a une valeur de pH capable de supprimer le blocage effectué par la première composition **(11).**
